# FASCICULE DE BREVET EUROPEEN

(11) **EP 0 676 451 B1**
(45) Date de publication et mention de la délivrance du brevet: **05.02.1997**
(21) Numéro de dépôt: 95400474.3
(22) Date de dépôt: 03.03.1995
(51) Int. Cl.: C08L 93/02, A61K 7/06, A61K 7/48, C08J 3/07

(54) **Dispersions aqueuses de résines, leur utilisation en cosmétique et compositions cosmétiques obtenues**
Wässrige Harzdispersionen, ihre Verwendung in der Kosmetik und daraus hergestellte kosmetische Zusammensetzungen
Aqueous dispersions of resins, their use in cosmetics and cosmetic compositions obtained therefrom

(30) Priorité: 07.04.1994 FR 9404113
(43) Date de publication de la demande: 11.10.1995
(73) Titulaire: L'OREAL, 75008 Paris (FR)
(72) Inventeur: Mougin, Nathalie, F-75011 Paris (FR); Mondet, Jean, F-93700 Drancy (FR)
(74) Mandataire: Dodin, Catherine

(56) Documents cités:
- EP-A- 0 530 084
- US-A- 5 053 218

## Description

La présente invention a trait à des compositions cosmétiques comprenant, à titre d'ingrédient nouveau, une dispersion aqueuse de résine, aussi appelée pseudo-latex.
Par pseudo-latex, on entend une dispersion constituée de particules généralement sphériques de polymère, dans une phase aqueuse appropriée.

Il est connu d'utiliser des pseudo-latex de polymères synthétiques dans les compositions cosmétiques. Ces polymères synthétiques sont généralement utilisés pour leurs propriétés intéressantes d'agent filmogène.
Il est ainsi connu, par exemple, d'utiliser des émulsions aqueuses d'homo et/ou copolymères synthétiques comme base pour vernis à ongles (JP04103511).
Il est également connu, par exemple par EP 568035, une composition contenant une émulsion aqueuse de polymères synthétiques, en association avec un plastifiant, pouvant être utilisée dans des produits cosmétiques tels que les vernis à ongles ou les produits de maquillage.
Les polymères utilisés jusqu'à présent étaient toutefois tous d'origine synthétique.
La demanderesse s'est posée le problème de pallier cet inconvénient en évitant d'utiliser des résines synthétiques dans ses produits cosmétiques, sans en altérer les qualités.
Les résines naturelles d'origine végétale ou animale constituent une classe intéressante de produits, de par leurs propriétés particulières liées à leur nature.
Il n'est toutefois pas manifeste que ces résines d'origine naturelles peuvent être utilisées telles quelles dans des compositions cosmétiques, étant donné, en particulier le fait qu'elles sont généralement insolubles dans l'eau, solvant particulièrement utilisé dans l'industrie des cosmétiques.
La présente invention a pour but de solutionner ce problème et de proposer des résines naturelles pour leur utilisation dans le domaine cosmétique, ces résines se présentant sous une forme adéquate, à savoir sous forme de dispersions aqueuses.

Un premier objet de la présente invention est donc une dispersion aqueuse de résine d'origine naturelle, seule ou en mélange, choisie dans le groupe constitué par la résine shellac, la gomme de sandaraque, les élémis, les dammars et les copals.
Les procédés de préparation de ces dispersions aqueuses de résines naturelles constituent un autre objet de la présente invention.
Enfin, leur utilisation dans le domaine cosmétique en général, et dans certaines applications en particulier, constitue un autre objet de l'invention.

L'invention présente comme avantage de permettre la réalisation de dispersions aqueuses de résines naturelles stables, c'est-à-dire ne présentant pas de trace de floculation, de coalescence et/ou de sédimentation après plusieurs jours de conservation.

De plus, l'utilisation d'un polymère sous forme de dispersion présente l'avantage de pouvoir travailler à concentration plus élevée, avec un temps de séchage beaucoup plus rapide, ceci étant dû au fait que l'on peut utiliser moins d'eau tout en ayant une concentration élevée en polymère filmogène sans que l'on ne rencontre de problème de viscosité, le polymère étant en dispersion dans la phase aqueuse et non en solution.

Dans la suite de la présente description, les pourcentages sont donnés en poids, sauf indication contraire.

Comme résine naturelle susceptible d'être mise en dispersion aqueuse dans l'optique de la présente invention, on cite la résine shellac, la gomme de sandaraque, les dammars, les élémis ou les copals.
La résine shellac est une sécrétion animale, composée principalement de résine et de cire, et est soluble dans certains solvants organiques.
La gomme de sandaraque est une résine qui peut être extraite de l'écorce d'arbres tels que le *thuya articulata* ou le *callitris verrucosa*.
Elle est composée principalement d'acides tels que l'acide pimarique, l'acide callitrolique et l'acide sandaricinique. Elle est insoluble dans l'eau mais peut être solubilisée dans des solvants organiques tels que l'éthanol, l'acétone ou l'éther.
Les dammars sont des résines provenant d'arbres des genres Damara ou Shoréa, et contiennent généralement 62,5% de résène (40% de solubles et 22,5% d'insolubles dans l'alcool) et 23 % d'acide.
Les élémis sont des résines provenant d'arbres du genre Icica et contiennent environ 30% d'amyrines diverses, 40% de résènes, ainsi que des acides et des huiles essentielles.
Les copals sont des résines extraites de diverses variétés de *Trachylobium verrucosum*, grands arbres de la famille des légumineuses semblables à des frênes.
Leurs constituants principaux sont les acides résiniques. On trouve également dans leur composition des résènes et des huiles essentielles.

D'une manière générale, les dispersions aqueuses de résines naturelles sont préparées de la manière suivante:
(a) on dissout la résine dans un solvant organique ;
(b) on ajoute de l'eau de manière à former une émulsion ;
(c) on stabilise ladite émulsion :
   (c₁) lorsque la résine ne comporte pas de groupement ionisable, en ajoutant un tensio-actif et en homogénéisant l'émulsion de manière à diminuer la taille des particules ; ou bien
   (c₂) lorsque la résine comporte des groupements ionisables, en neutralisant de 20 à 80% des groupements ionisables de la résine de manière à auto-stabiliser l'émulsion ;
(d) puis on évapore le solvant organique.
Il est nécessaire de stabiliser l'émulsion obtenue avant l'évaporation du solvant organique.

On peut le faire en ajoutant des tensioactifs, par exemple lorsque la résine naturelle ne comporte pas des groupements ionisables, tels que des groupements -COOH, -SO₃H ou amine tertiaire. Les tensioactifs ont alors également pour fonction de stabiliser la dispersion aqueuse obtenue.

On peut utiliser des tensioactifs cationiques ou amphotères, mais de préférence des tensioactifs non ioniques tels qu'un copolymère polyoxyéthylène/polyoxypropylène, ou anioniques tels que le laurylsulfate de sodium.
On peut également stabiliser l'émulsion en sous-neutralisant la résine, c'est-à-dire en ne la neutralisant que partiellement, ce qui va provoquer l'auto-stabili-sation de l'émulsion. Ceci est possible lorsque la résine comporte des groupements ionisables, comme cela est le cas de la résine shellac.
La sous-neutralisation peut être effectuée par addition d'un agent monobasique non volatil, tel qu'une base minérale comme la soude ou la potasse, ou qu'un aminoalcool pris dans le groupe constitué par l'amino-2-méthyl-2-propanol-1 (AMP), la triéthanolamine, la triisopropanolamine (TIPA), la monoéthanolamine, la diéthanolamine, la tri-[(hydroxy-2)-propyl-1]-amine, I'amino-2-méthyl-2-propanediol-1,3 (AMPD) et l'amino-2 hydroxyméthyl-2 propanediol-1,3.
On neutralise 20 à 80% des groupements ionisables de la résine pour stabiliser la dispersion aqueuse sans solubiliser le polymère.

Selon un premier mode de préparation de dispersions aqueuses d'origine naturelle selon l'invention, on prépare une dispersion aqueuse de résine shellac par dissolution de la résine dans un solvant organique, addition d'eau de manière à former une émulsion, neutralisation partielle de la résine de manière à auto-stabiliser l'émulsion puis évaporation du solvant organique.
Ce procédé de préparation s'effectue donc en l'absence de tensioactifs comme stabilisant externes.
Selon un second mode de préparation de dispersions aqueuses d'origine naturelle selon l'invention, on prépare une dispersion aqueuse de gomme de sandaraque par dissolution de la résine dans un solvant organique, addition d'eau contenant un tensioactif stabilisant, de manière à former une émulsion stable, puis homogénéisation de l'émulsion de manière à diminuer la taille des particules, et évaporation du solvant.
On peut ainsi préparer des dispersions aqueuses de différentes résines naturelles, selon que ces résines comportent ou non des groupements ionisables.

Selon la nature de la résine et l'utilisation ultérieure de la dispersion, il peut être nécessaire d'ajouter à ladite dispersion aqueuse un agent plastifiant.
Cet agent peut être hydrosoluble ou insoluble dans l'eau, et doit être apte à solubiliser la résine.
On peut l'ajouter soit directement dans la dispersion aqueuse déjà préparée, soit pendant l'étape de mise en émulsion, avant évaporation du solvant.
Parmi les agents plastifiants pouvant être utilisés dans la présente invention, on peut citer:
- les Carbitols de la Société Union Carbide à savoir le Carbitol ou diéthylène glycol éthyléther, le méthyl Carbitol ou diéthylène glycol méthyléther, le butyl Carbitol ou diéthylène glycol butyléther ou encore l'hexyl Carbitol ou diéthylène glycol hexyléther,
- les Cellosolves de la Société Union Carbide à savoir le Cellosolve ou éthylène glycol éthyléther, le butyl Cellosolve ou éthylène glycol butyléther, l'hexyl Cellosolve ou éthylène glycol hexyléther,
- les dérivés de propylène glycol et en particulier le propylène glycol phényléther, le propylène glycol diacétate, le dipropylène glycol butyléther, le tripropylène glycol butyléther, ainsi que les Dowanols de la Société Dow Chemical tels que le Dowanol PM ou propylène glycol méthyléther, le Dowanol DPM ou dipropylène glycol éthyléther, le Dowanol TPM ou tripropylène glycol méthyléther et le Dowanol DM ou diéthylène glycol méthyléther,
- l'alcool benzylique,
- le citrate de triéthyle,
- le 1,3-butylène glycol,
- les phtalates et adipates de diéthyle, de dibutyle et de diisopropyle,
- les tartrates de diéthyle et de dibutyle,
- les phosphates de diéthyle, de dibutyle et de diéthyl-2-hexyle, et
- les esters de glycérol tels que le diacétate de glycérol (diacétine) et le triacétate de glycérol (triacétine).
On introduit généralement 5 à 40 % d'agent plastifiant par rapport au poids de matière sèche de résine.

Les dispersions aqueuses ainsi obtenues peuvent être utilisées dans toutes les applications, entre autre cosmétiques, nécessitant l'emploi de pseudo-latex, en remplacement, partiel ou total, des pseudo-latex de résines synthétiques employés auparavant.
On a en effet découvert que, de manière surprenante, ces dispersions aqueuses de résines naturelles présentaient de bonnes propriétés filmogènes, comparables à celles des pseudo-latex synthétiques habituellement utilisés.
La formulation des compositions cosmétiques et leurs procédés de préparation sont à optimiser par l'homme du métier sur base de ses connaissances générales techniques.
En particulier, on peut employer ces dispersions aqueuses dans la réalisation de produits capillaires, tels que des lotions ou des mousses de coiffage, ou des laques, ou encore dans la réalisation de produits de maquillage, tels que le mascara et le vernis à ongles, ou encore dans les produits de soin tels que les masques de beauté pour le visage ou les soins pour les ongles.
On peut ajouter aux compositions cosmétiques comprenant une dispersion aqueuse selon l'invention des adjuvants couramment utilisés dans l'industrie cosmétique, tels que des épaississants, des filtres UV, des parfums, des charges, des colorants et des pigments cosmétiques, en une quantité de l'ordre de 0,01-2%.

Il est également possible d'ajouter des actifs, en une quantité de 0,01-0,5%, parmi lesquels on peut citer les vitamines et leurs dérivés; les matières biologiques et leurs dérivés tels que la kératine, les protéines, les hydrolysats, le chitosane, la mélanine, l'acide hyaluronique, les oligo-éléments et le collagène; la glycérine; des phospholipides.

L'invention est illustrée plus en détail par les exemples suivants, qui décrivent deux procédés de préparation de dispersions aqueuses de résine shellac et de gomme de sandaraque (exemples 1 et 2) et des exemples d'utilisation de ces dispersions aqueuses dans des compositions cosmétiques (exemples 3 à 7).

Exemple 1

On dissout 100 g de résine shellac (gomme laque 55 Astra de KPS), présentant un indice d'acide de 74, dans un mélange de 320 g de méthyléthylcétone et de 80 g d'isopropanol, puis l'on ajoute 9,37 g d'amino-2-méthyl-2-propanol afin de neutraliser environ 80% des groupes -COOH de la résine shellac.
On agite le mélange ainsi obtenu à l'aide d'un disperseur du type Moritz, à 2500 tours par minute, puis on introduit, en 15 minutes, une phase aqueuse comprenant 5 g de monométhyléther de tripropylène glycol dans 390,6 ml d'eau permutée.
On obtient ainsi une émulsion auto-stabilisée que l'on peut agiter pendant 15 minutes à une vitesse de 3000 tours par minute pour terminer la stabilisation.
On élimine alors les solvants organiques par évaporation à 50°C sous pression réduite et l'on obtient ainsi 505 g de dispersion aqueuse de shellac, d'aspect laiteux, présentant les caractéristiques suivantes:
- taux d'extrait sec total : 24%
- pH : 7,5
- viscosité à 20°C (Contraves) : 1,5 mPa.s
- diamètre moyen des particules: 90 nm
- polydispersité en taille des particules: 0,20 (mesurée par diffusion quasi-élastique de la lumière, à l'aide d'un appareil du type COULTER N4)

### Exemple 2

On mélange 1390 g de tétrahydrofurane et 48 g de monométhyléther de tripropylèneglycol, puis on ajoute sous agitation 160 g de gomme de sandaraque ("gomme de sandaraque en larmes" de EMIGA), présentant un indice d'acide de 136.
On filtre la solution obtenue puis on l'agite à l'aide d'un disperseur du type Moritz, à 2500 tours par minute et on y introduit, en 20 minutes, une phase aqueuse contenant 66 g d'hydroxyde de sodium, 3,2 g de laurylsulfate de sodium et 1320 ml d'eau permutée.
On homogénéise l'émulsion ainsi obtenue, de manière à réduire la taille des globules gras, sur un homogénéisateur haute pression à 700 bars, en faisant deux passages.
On élimine ensuite les solvants organiques par évaporation à 40°C sous pression réduite, puis l'on concentre l'émulsion jusqu'à obtention d'un extrait sec de 14% environ.
On obtient ainsi une dispersion aqueuse de gomme de sandaraque, présentant les caractéristiques suivantes:
- taux d'extrait sec total : 14%
- diamètre moyen des particules : 300 nm
- polydispersité en taille : 0,1 (mesurée par diffusion quasi-élastique de lumière à l'aide d'un appareil du type COULTER N4).

### Exemple 3

On prépare une laque aérosol pour cheveux en conditionnant, dans un récipient aérosol approprié:
. dispersion aqueuse de résine shellac (exemple 1) 85,7 g
. eau déminéralisée q.s.p. 100 g
On pressurise par addition de 30 g de diméthyléther pour 70 g de mélange.
On obtient ainsi une laque présentant les caractéristiques suivantes: bonne élimination au brossage, bonne élimination au shampooing et bon niveau de démêlage

### Exemple 4

On prépare une lotion de coiffage comprenant
. dispersion aqueuse de résine shellac (exemple 1) 75 g
. parfum, colorant et conservateur q.s.
. eau déminéralisée q.s.p. 100 g
Cette lotion, qui peut éventuellement être présentée en flacon-pompe, apporte du maintien à la chevelure après application sur cheveux mouillés ou sur cheveux secs.

### Exemple 5

On prépare une mousse de coiffage aérosol ayant la composition suivante:
. dispersion aqueuse de résine shellac (exemple 1) 25 g
. parfum, colorant et conservateur q.s.
. eau déminéralisée q.s.p. 100 g
On introduit 90 g de la composition obtenue dans un boîtier aérosol sans tube plongeur. On procède à la fixation de la valve et à la fermeture hermétique du récipient, puis on introduit 10 g d'un mélange propulseur butane/iso-butane/propane (3,2 bars).
La mousse de coiffage obtenue présente de bonnes caractéristiques cosmétiques.

### Exemple 6

On prépare un mascara ayant la composition suivante :
- partie A 11,5 g de stéarate de triéthanolamine
   7,0 g de cire d'abeilles
   4,1 g de cire de Carnauba
   11,4 g de paraffine
- partie B 5,5 g d'oxyde de fer noir
- partie C 4,5 g de gomme arabique
   0,16 g d'hydroxy-éthyl-cellulose, Cellosize QP (Amerchol)
- partie D 3,0 g de dispersion aqueuse de résine shellac de l'exemple 1
- conservateurs q.s.
- eau q.s.p. 100,0 g
Le mascara est préparé en portant les ingrédients de la partie A à 85°C, puis en ajoutant la partie B et en agitant avec une turbine. On fait ensuite bouillir l'eau de la préparation, on ajoute les conservateurs, puis, à 85°C, les ingrédients de la partie C. On ajoute alors la phase aqueuse obtenue à 85°C, au mélange des parties A+B à 80°C, sous agitation à l'aide d'une turbine (émulsification à 30°C), puis on ajoute la partie D et l'on mélange à l'aide d'une pale.
On obtient ainsi un mascara crème qui s'applique facilement et qui présente des qualités d'adhérence intéressantes.

### Exemple 7

On prépare, de la même manière que précédemment, un mascara crème, de composition suivante:
- partie A 11,5 g de stéarate de triéthanolamine
   7,0 g de cire d'abeilles
   4,1 g de cire de Carnauba
   11,4 g de paraffine
- partie B 5,0 g d'oxyde de fer noir
- partie C 4,5 g de gomme arabique
   0,16 g d'hydroxy-éthyl-cellulose, Cellosize QP (Amerchol)
   2,0 g d'hydrolysat de kératine, Kérasol (Croda)
- partie D 2,0 g de dispersion aqueuse de gomme de sandaraque (ex.2)
- conservateurs q.s.
- eau q.s.p. 100, 0 g
On obtient ainsi un mascara crème qui s'applique facilement et qui présente des qualités d'adhérence intéressantes.

### Exemple 8

On prépare un soin des ongles ayant la composition suivante, par simple mélange et agitation :
. dispersion aqueuse de résine shellac (exemple 1) 91,2 g
. glycérine 3 g
. protéine de calcium 0,3 g
. hydrolysat de blé 0,2 g
. dérivé de chitosane 0,3 g
. eau 5g
Cette composition est applicable aisément sur l'ongle et permet l'obtention, après séchage, d'un film sec et brillant, qui s'élimine facilement à l'eau.
L'application quotidienne de cette composition sur l'ongle permet, après plusieurs semaines, d'améliorer l'état général des ongles.

## Revendications

1. Procédé de préparation d'une dispersion aqueuse stable de particules sphériques constituées de résine d'origine naturelle, seule ou en mélange, choisie dans le groupe constitué par la résine shellac, la gomme de sandaraque, les élemis, les dammars,et les copals, caractérisé par le fait qu'il comprend les étapes suivantes :
(a) on dissout la résine dans un solvant organique ;
(b) on ajoute de l'eau de manière à former une émulsion ;
(c) on stabilise ladite émulsion :
(c₁) lorsque la résine ne comporte pas de groupement ionisable, en ajoutant un tensio-actif et en homogénéisant l'émulsion de manière à diminuer la taille des particules ; ou bien
(c₂) lorsque la résine comporte des groupements ionisables, en neutralisant de 20 à 80% des groupements ionisables de la résine de manière à auto-stabiliser l'émulsion ;
(d) puis on évapore le solvant organique.

2. Procédé de préparation d'une dispersion aqueuse stable de particules sphériques constituées de résine shellac, dans lequel on dissout la résine dans un solvant organique, on ajoute de l'eau de manière à former une émulsion, on neutralise de 20 à 80% des groupements ionisables de la résine de manière à auto-stabiliser l'émulsion puis on évapore le solvant organique.

3. Procédé de préparation d'une dispersion aqueuse stable de particules sphériques constituées de gomme de sandaraque, dans lequel on dissout la gomme dans un solvant organique, on ajoute de l'eau de manière à former une émulsion et un tensioactif pour stabiliser l'émulsion, puis on homogénéise l'émulsion de manière à diminuer la taille des particules, et l'on évapore le solvant.

4. Dispersion aqueuse stable de particules sphériques constituées de résine d'origine naturelle, seule ou en mélange, choisie dans le groupe constitué par la résine shellac, la gomme de sandaraque, les élemis, les dammars,et les copals, caractérisée par le fait qu'elle est susceptible d'être obtenue par le procédé selon l'une quelconque des revendications 1 à 3.

5. Dispersion aqueuse stable de particules sphériques constituées de résine shellac susceptible d'être obtenue par le procédé selon la revendication 2.

6. Dispersion aqueuse stable de particules sphériques constituées de gomme de sandaraque, susceptible d'être obtenue par le procédé selon la revendication 3.

7. Utilisation d'une dispersion aqueuse stable de particules sphériques de résine naturelle telle que définie dans l'une quelconque des revendications 4 à 6 dans et pour la préparation d'une composition cosmétique.

8. Utilisation selon la revendication 7, dans un produit capillaire, un produit de maquillage et/ou dans une base de soin pour les ongles.

9. Composition cosmétique comprenant une dispersion aqueuse selon la revendication 4 à 6.

10. Composition selon la revendication 9, se présentant sous forme d'un produit capillaire tel qu'une laque aérosol, une lotion de coiffage, une mousse de coiffage; d'un produit de maquillage tel qu'un mascara ou un vernis à ongles; d'un produit de soin tel qu'un masque de beauté pour le visage ou une base de soin pour ongles.

## Patentansprüche

1. Verfahren zur Herstellung einer stabilen wäßrigen Dispersion von kugelförmigen Partikeln, die aus einem Harz natürlichen Ursprungs bestehen, das alleine oder im Gemisch unter Schellack, Sandarakgummi, Elemi, Dammarharzen und Kopalen ausgewählt ist, dadurch gekennzeichnet, daß es die folgenden Schritte umfaßt:
(a) Lösen des Harzes in einem organischen Lösungsmittel;
(b) Zusatz von Wasser, um eine Emulsion zu bilden;
(c) Stabilisierung der Emulsion;
(c₁) Zusatz eines grenzflächenaktiven Stoffes und Homogenisierung der Emulsion, so daß die Teilchengröße vermindert wird, wenn das Harz keine ionisierbaren Gruppen enthält; oder
(c₂) Neutralisation von 20 bis 80 % der ionisierbaren Gruppen des Harzes, so daß die Emulsion autostabilisiert wird, wenn das Harz ionisierbare Gruppen enthält; und
(d) anschließendes Verdampfen des organischen Lösungsmittels.

2. Verfahren zur Herstellung einer stabilen wäßrigen Dispersion von kugelförmigen Partikeln, die aus Schellack bestehen, in dem das Harz in einem organischen Lösungsmittel gelöst wird, Wasser zugesetzt wird, um eine Emulsion zu bilden, 20 bis 80 % der ionisierbaren Gruppen des Harzes neutralisiert werden, so daß die Emulsion autostabilisiert wird, und anschließend das organische Lösungsmittel verdampft wird.

3. Verfahren zur Herstellung einer stabilen wäßrigen Dispersion von kugelförmigen Partikeln, die aus Sandarakgummi bestehen, in dem das Gummi in einem organischen Lösungsmittel gelöst wird, Wasser zur Bildung einer Emulsion und ein grenzflächenaktiver Stoff zur Stabilisierung der Emulsion zugegeben wird, worauf die Emulsion homogenisiert wird, um die Teilchengröße zu vermindern, und das Lösungsmittel verdampft wird.

4. Stabile wäßrige Dispersion von kugelförmigen Partikeln, die aus einem Harz natürlichen Ursprungs bestehen, das alleine oder im Gemisch unter Schellack, Sandarakgummi, Elemi, Dammarharzen und Kopalen ausgewählt ist, dadurch gekennzeichnet, daß sie nach dem Verfahren nach einem der Ansprüche 1 bis 3 hergestellt werden kann.

5. Stabile wäßrige Dispersion von kugelförmigen Partikeln, die aus Schellack bestehen, die nach dem Verfahren nach Anspruch 2 hergestellt werden kann.

6. Stabile wäßrige Dispersion von kugelförmigen Partikeln, die aus Sandarakgummi bestehen, die nach dem Verfahren nach Anspruch 3 hergestellt werden kann.

7. Verwendung einer stabilen wäßrigen Dispersion von kugelförmigen Partikeln von natürlichen Harzen nach einem der Ansprüche 4 bis 6 in einer kosmetischen Zusammensetzung und zur Herstellung einer kosmetischen Zusammensetzung.

8. Verwendung nach Anspruch 7 in einem Produkt für das Haar, einem Produkt zum Schminken und/oder einer Pflegegrundlage für die Nägel.

9. Kosmetische Zusammensetzung, die eine wäßrige Dispersion nach einem der Ansprüche 4 bis 6 enthält.

10. Zusammensetzung nach Anspruch 9, die in Form eines Produkts für das Haar, wie einem Aerosolhaarlack, einer Frisierlotion und einem Frisierschaum, in Form eines Produkts zum Schminken, wie Mascara oder Nagellack, in Form eines Pflegeproduktes, wie einer Schönheitsmaske für das Gesicht oder einer Pflegegrundlage für die Nägel, vorliegt.

## Claims

1. Process for preparing a stable aqueous dispersion of spherical particles consisting of a resin of natural origin, alone or in the form of a mixture, chosen from the group consisting of shellac resin, sandarac gum, elemis, dammars and copals, characterized in that it comprises the following steps:
(a) the resin is dissolved in an organic solvent;
(b) water is added so as to form an emulsion;
(c) the said emulsion is stabilized:
(c₁) when the resin does not contain an ionizable group, by adding a surfactant and by homogenizing the emulsion so as to reduce the size of the particles; or alternatively
(c₂) when the resin does contain ionizable groups, by neutralizing from 20 to 80% of the ionizable groups of the resin so as to self-stabilize the emulsion;
(d) the organic solvent is then evaporated.

2. Process for preparing a stable aqueous dispersion of spherical particles consisting of shellac resin, in which the resin is dissolved in an organic solvent, water is added so as to form an emulsion, 20 to 80% of the ionizable groups of the resin are neutralized so as to self-stabilize the emulsion and then the organic solvent is evaporated.

3. Process for preparing a stable aqueous dispersion of spherical particles consisting of sandarac gum, in which the gum is dissolved in an organic solvent, water is added so as to form an emulsion and a surfactant is added so as to stabilize the emulsion, and then the emulsion is homogenized so as to reduce the size of the particles and the solvent is evaporated.

4. Stable aqueous dispersion of spherical particles consisting of a resin of natural origin, alone or in the form of a mixture, chosen from the group consisting of shellac resin, sandarac gum, elemis, dammars and copals, characterized in that it is capable of being obtained by the process according to any one of Claims 1 to 3.

5. Stable aqueous dispersion of spherical particles consisting of shellac resin, capable of being obtained by the process according to Claim 2.

6. Stable aqueous dispersion of spherical particles consisting of sandarac gum, capable of being obtained by the process according to Claim 3.

7. Use of a stable aqueous dispersion of spherical particles of natural resin as defined in any one of Claims 4 to 6 in and for the preparation of a cosmetic composition.

8. Use according to Claim 7, in a hair product, a make-up and/or in a nail treatment base.

9. Cosmetic composition comprising an aqueous dispersion according to Claim 4 to 6.

10. Composition according to Claim 9, provided in the form of a hair product such as an aerosol lacquer, a hair-styling lotion, a hair-styling foam; of a make-up such as a mascara or a nail varnish; of a treatment product such as a face mask or a nail treatment base.
